# FASCICULE DE BREVET EUROPEEN

(11) **EP 3 967 624 B1**
(45) Date de publication et mention de la délivrance du brevet: **21.01.2026**
(21) Numéro de dépôt: 21196261.8
(22) Date de dépôt: 13.09.2021
(51) Int. Cl.: B65D 71/70, B65D 1/02

(54) **PLATEAU DE LIVRAISON ET SYSTEME D´ EMBALLAGE D´ ELEMENTS MEDICAUX**
LIEFERPLATTE UND VERPACKUNGSSYSTEM FÜR MEDIZINISCHE ELEMENTE
DELIVERY TRAY AND PACKAGING SYSTEM FOR MEDICAL ITEMS

(30) Priorité: 11.09.2020 FR 2009215
(43) Date de publication de la demande: 16.03.2022
(73) Titulaire: A. Raymond et Cie, 38000 Grenoble (FR)
(72) Inventeur: REY, Gaëtan, 38500 VOIRON (FR)
(74) Mandataire: IP Trust

(56) Documents cités:
- EP-A1- 0 285 496
- EP-A1- 0 521 705
- EP-A1- 0 610 100
- EP-A1- 2 753 550
- WO-A1-01/17682
- WO-A1-2017/136667
- WO-A2-2009/126945
- CA-A1- 2 215 561
- FR-A- 1 569 978
- FR-A1- 2 679 878
- GB-A- 2 257 121
- GB-A- 2 537 637
- US-A- 3 802 592
- US-A- 6 007 779
- US-A1- 2002 114 737
- US-B2- 9 586 722

## Description

### DOMAINE DE L'INVENTION

La présente invention concerne un plateau et un système d'emballage pour conditionner, livrer, et distribuer des éléments médicaux. Il concerne également un procédé d'emballage d'éléments médicaux. L'invention concerne plus particulièrement le conditionnement, en vue de leur distribution, de bouchons destinés à refermer des flacons médicaux.

### ARRIERE PLAN TECHNOLOGIQUE DE L'INVENTION

De tels bouchons sont généralement livrés en vrac et versés dans un bol vibrant, ou simplement disposés dans un plateau, pour ensuite être placés, à la main ou automatiquement par une machine, un par un, sur un flacon pour le fermer.

Ces solutions de livraison ne sont pas adéquates lorsque les bouchons sont destinés à refermer des flacons destinés à un usage médical. Dans ce domaine, les bouchons et les flacons doivent être dépourvus de toute contamination, notamment particulaire. Or, si aucune précaution particulière n'est prise, les contacts et frottements des bouchons entre eux ou des bouchons contre les surfaces du plateau ou du bol vibrant, sont susceptibles de générer des particules qui peuvent alors se retrouver sur les bouchons, puis, dans les flacons. Cela peut avoir des conséquences très graves lorsque le flacon est utilisé pour contenir une solution médicale destinée à être prélevée par une seringue, les particules pouvant alors se retrouver dans la seringue et ensuite être injectées dans l'organisme d'un patient.

Il est donc important de disposer d'une solution pour conditionner et livrer des bouchons de flacons, et plus généralement des éléments médicaux ou articles de conditionnement, permettant de limiter la génération de particules.

On connaît des documents EP2753550, US10064787 et US9586722, qui divulgue un plateau de livraison selon le préambule de la revendication 1, des plateaux et systèmes d'emballages permettant de stocker individuellement différents types d'éléments médicaux (seringues, flacons). Ces éléments médicaux ne peuvent pas se contacter et la génération de particules est ainsi réduite. EP2753550 cherche plus particulièrement à réduire la surface de contact entre les éléments médicaux et la surface d'un plateau pour réduire les zones de frottement.

### OBJET DE L'INVENTION

Un but de l'invention est de proposer un plateau, un système d'emballage, ainsi qu'un procédé d'emballage se distinguant et améliorant cet état de la technique.

### BREVE DESCRIPTION DE L'INVENTION

En vue de la réalisation de ce but, l'objet de l'invention propose un plateau de livraison d'éléments médicaux, le plateau comprenant une face inférieure et une face supérieure, la face supérieure présentant une pluralité de logements destinés chacun à recevoir un unique élément médical.

Selon l'invention la face inférieure du plateau présente une pluralité de capuchons, les capuchons étant disposés et dimensionnés de sorte que, lorsque le plateau est empilé sur un second plateau identique, chaque capuchon du plateau soit apte à refermer un logement du second plateau.

Selon d'autres caractéristiques avantageuses et non limitatives de l'invention, prises seules ou selon toute combinaison techniquement réalisable :
- chaque logement est délimité latéralement par une cloison perpendiculaire à la face supérieure du plateau ;
- chaque capuchon est délimité latéralement par une paroi perpendiculaire à la face inférieure du plateau ;
- les capuchons du plateau sont respectivement disposés au droit des logements dudit plateau, dit premier plateau, et sont respectivement dimensionnés pour refermer les logements d'un second plateau identique et sous-jacent au premier plateau, par emboitement ou par aboutement des cloisons des logements du second plateau et des parois des capuchons en vis-à-vis du premier plateau ;
- les logements présentent chacun une surface interne, la surface interne des logements étant munie d'au moins un élément de maintien latéral pour maintenir et limiter les mouvements latéraux de l'élément médical dans le logement ;
- chaque logement présente un fond plein, formé par la face supérieure du plateau, et chaque capuchon présente un fond plein, formé par la face inférieure du plateau ;
- les capuchons sont munis chacun d'un élément de maintien vertical pour maintenir et limiter le mouvement vertical

d'un élément médical disposé dans le logement du second plateau sur lequel le plateau a été empilé ;
- chaque logement présente un fond ajouré, et chaque capuchon présente un fond ajouré, du fait de la présence d'un orifice dans le plateau, centré sur le fond de chaque logement, l'orifice associé à un logement présentant des dimensions inférieures aux dimensions d'un élément médical destiné à occuper ledit logement.
- une partie supérieure des logements est déformable.

Selon un autre aspect, l'objet de l'invention propose un système d'emballage pour éléments médicaux comprenant :
- une cuve ayant une ouverture, un fond et une paroi périphérique ;
- un empilement formé d'une pluralité de plateaux tels que décrits précédemment, l'empilement étant placé dans la cuve ; et
- un opercule poreux scellé sur un bord supérieur de la paroi périphérique de la cuve pour la refermer.

Selon d'autres caractéristiques avantageuses et non limitatives de l'invention, prises seules ou selon toute combinaison techniquement réalisable :
- le système d'emballage comprend en outre un couvercle disposé sur l'empilement de plateaux pour refermer les logements d'un plateau supérieur de l'empilement ;
- le couvercle est une plaque rigide munie sur une face inférieure d'une pluralité de capuchons aptes à refermer les logements du plateau supérieur de l'empilement ;
- le couvercle est composé d'une pluralité de bandes munies de capuchons aptes à refermer les logements du plateau supérieur de l'empilement ;
- les bandes et les capuchons sont formées d'un matériau flexible ;
- chaque bande comprend des zones de flexion formées entre chaque capuchon ;
- les logements d'un plateau supérieur de l'empilement sont refermés par des bouchons unitaires ;
- le fond de la cuve est muni de bossages configurés pour s'emboiter dans des capuchons d'un plateau inférieur de l'empilement ;
- la cuve est placée, sous vide, dans au moins une sache étanche.

Selon encore un autre aspect, l'objet de l'invention propose un procédé d'emballage d'éléments médicaux comprenant les étapes suivantes :
- fournir une cuve présentant une ouverture, un fond et une paroi périphérique ;
- disposer un plateau inférieur tel que décrit précédemment au fond de la cuve ;
- disposer au moins un plateau supplémentaire tel que décrit précédemment pour former un empilement de plateaux dans la cuve, les capuchons du plateau supplémentaire refermant les logements d'un plateau directement sous-jacent ;
- sceller un opercule poreux sur un bord supérieur de la paroi périphérique de la cuve ;
- placer la cuve dans au moins une sache étanche, et mettre la sache sous vide.

Selon d'autres caractéristiques avantageuses et non limitatives de l'invention, prises seules ou selon toute combinaison techniquement réalisable :
- l'opercule est rendu solidaire de la cuve par soudage de l'opercule sur le bord supérieur de la paroi périphérique de la cuve ;
- le procédé d'emballage comprend une étape visant à disposer un couvercle sur un plateau supérieur de l'empilement ;
- l'opercule est également scellé au couvercle.

### BREVE DESCRIPTION DES FIGURES

D'autres caractéristiques et avantages de l'invention ressortiront de la description détaillée de l'invention qui va suivre en référence aux figures annexées sur lesquels :
[Fig. 1] La figure 1 représente une vue en coupe d'un emballage conforme à l'invention ;
[Fig. 2] La figure 2 représente un plateau de livraison conforme à l'invention ;
[Fig. 3] La figure 3 représente les interactions de l'empilement de deux plateaux au niveau d'un logement conforme à l'invention ;
[Fig. 4a]
[Fig. 4b]

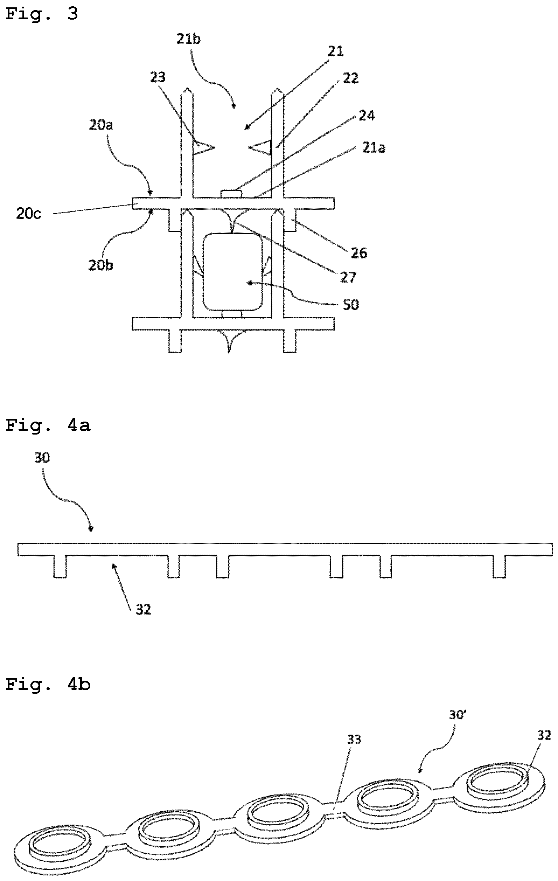

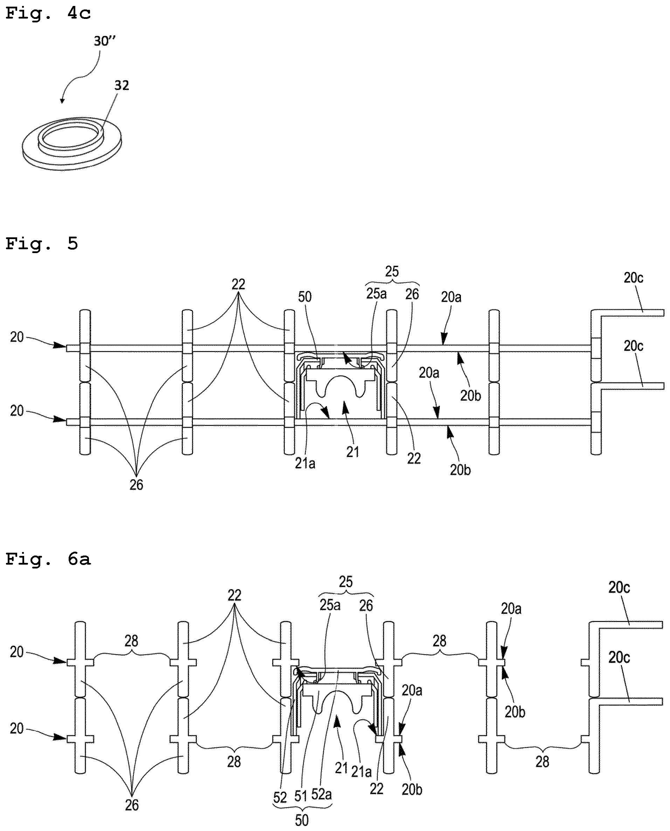

[Fig. 4c] Les figures 4a,4b et 4c représentent respectivement un couvercle conforme à un premier, deuxième et troisième mode de réalisation ;
[Fig. 5]
[Fig. 6a]

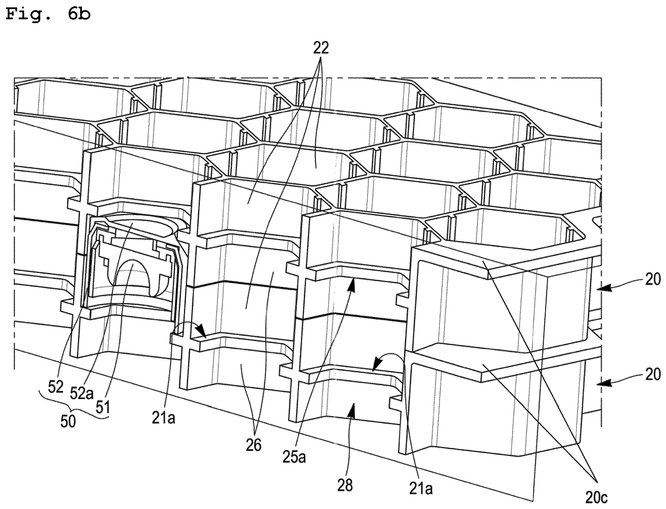

[Fig. 6b] Les figures 5, 6a et 6b représentent deux plateaux de livraison, empilés, conformes à l'invention ; les logements de plateaux présentent un fond plein (figure 5) ou un fond ajouré (figures 6a et 6b).

### DESCRIPTION DETAILLEE DE L'INVENTION

### Présentation générale du système d'emballage

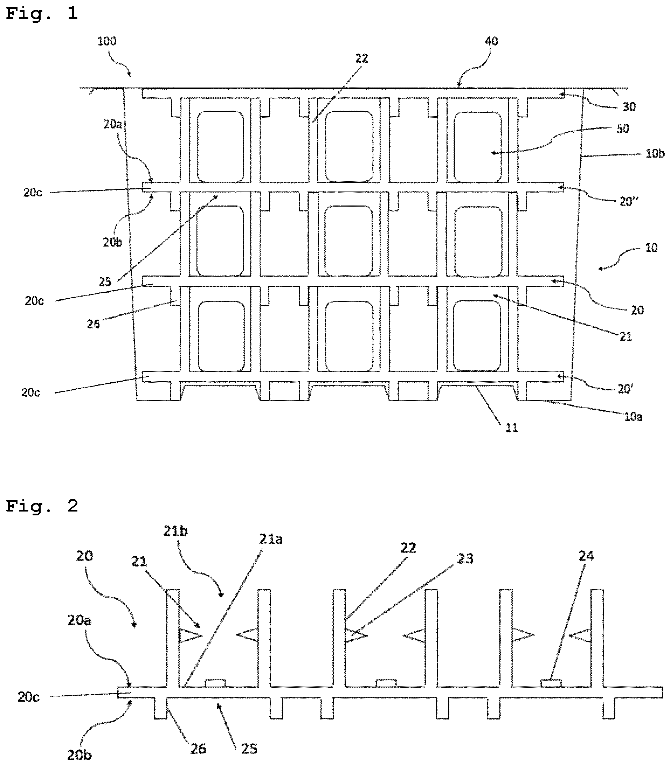

Comme cela est représenté sur la figure 1, un système d'emballage 100 conforme à l'invention comprend une cuve 10, un empilement formé d'une pluralité de plateaux de livraison 20 identiques, chacun muni de logements 21, empilés les uns sur les autres parallèlement au fond 10a de la cuve 10, ainsi qu'un opercule poreux 40 disposé sur l'ouverture de la cuve 10 pour la refermer. Le système d'emballage 100 peut également comprendre un couvercle 30 disposé sur le plateau supérieur 20" de l'empilement. La cuve 10 contenant les plateaux 20, et refermée par l'opercule poreux 40, est destinée à être placée dans au moins une sache étanche à l'air (et généralement deux de ces saches) dans laquelle on a évacué l'air pour placer l'ensemble sous vide.

Par souci de clarté, on précise que, dans la suite de cette description, le terme « plateau supérieur 20'' » désigne le plateau 20 de l'empilement situé le plus loin du fond 10a de la cuve 10 et le terme « plateau inférieur 20' » le plateau situé le plus proche du fond 10a.

Chacun des plateaux 20 accueille une pluralité d'éléments médicaux 50 stockés de manière individuelle dans un des logements 21 du plateau 20 sans qu'aucun contact ne soit possible entre eux. Par éléments médicaux 50, on désigne tout type d'élément à usage médical devant rester stérile et/ou propre et dépourvu de toute contamination particulaire. Il s'agit dans les exemples décrits et représentés de bouchons destinés à refermer des flacons mais il pourrait tout aussi bien s'agir de tout autre type d'élément médical, tel que des seringues ou des flacons. On adaptera bien entendu la forme et le volume des logements 21 pour que ces derniers puissent accueillir l'élément médical 50 en question. Comme cela sera rendu apparent dans la suite de cette description, l'empilement des plateaux 20 dans la cuve 10 permet de refermer les logements 21 et d'isoler les éléments médicaux 50 qui y sont placés afin de limiter le risque de contamination particulaire.

On note à ce propos que le plateau supérieur 20'' de l'empilement peut ne pas être exploité pour y placer des éléments médicaux 50. Ce plateau supérieur 20'', dans ce cas, a pour unique fonction de refermer les logements du plateau de l'empilement qui lui est directement sous-jacent. Alternativement à cette possibilité, et comme on l'a déjà évoqué, on peut prévoir de disposer un couvercle 30 sur le plateau supérieur 20'' de l'empilement pour en refermer les logements. Ce couvercle 30 peut prendre diverses formes qui seront présentées dans une section ultérieure de cette description.

### Description de la cuve

La cuve 10 est un élément creux de conditionnement destiné à recevoir les plateaux 20 dans lesquels sont disposés les éléments médicaux 50. La cuve 10 comprend une ouverture, un fond 10a et une paroi périphérique 10b délimitant ainsi sa forme générale. La paroi périphérique 10b peut être munie d'un épaulement permettant la manutention de la cuve 10, notamment par des équipements automatiques. Avantageusement, la cuve 10 présente une forme parallélépipédique pour optimiser l'espace nécessaire pour stocker un nombre donné d'éléments médicaux 50. Les dimensions de la cuve 10 sont choisies en fonction du nombre d'éléments médicaux 50 que l'on cherche à conditionner. Ces dimensions peuvent être conformes à une norme ou à un standard de manière à faciliter son exploitation à l'échelle industrielle. La cuve 10 peut être formée d'une matière plastique, par exemple à base de polypropylène, de polyéthylène téréphtalate amorphe, ou d'un polymère styrénique comme le polystyrène.

Dans l'exemple représenté, le fond 10a de la cuve 10 est muni de bossages 11 permettant le calage et/ou le centrage du plateau inférieur 20', par exemple par emmanchement de ces bossages sur des reliefs disposés sur la surface inférieure du plateau 20'. Ces reliefs peuvent par exemple constituer des capuchons dont on fera une description détaillée dans une section suivante de la présente description. Le plateau inférieur 20' (ainsi que tout l'empilement de plateaux) est ainsi immobilisé dans la cuve 10, les frottements entre le plateau 20' et la cuve 10 sont limités et on évite de la sorte la génération de particules.

Le fond 10a de la cuve 10 peut éventuellement comprendre des plots d'ajustement d'altitude (non représentés) sur lesquels viendra reposer le plateau inférieur 20'. Ces plots permettent d'ajuster la hauteur des empilements de plateaux 20 de manière à ce que le plateau supérieur 20" (ou le couvercle 30, s'il est présent) affleure toujours le niveau de l'ouverture de la cuve 10 et de l'opercule 40 de scellement, quelle que soit la hauteur unitaire des plateaux 20 ; en effet, cette hauteur unitaire peut varier selon la taille des éléments médicaux 50.

### Description des plateaux de livraison

L'empilement de plateaux 20 de la figure 1 est composé d'une pluralité de plateaux, tous identiques entre eux, et dont on a représenté un exemplaire conforme à l'invention sur la figure 2. Chaque plateau 20 comprend une face supérieure 20a et une face inférieure 20b.

La face supérieure 20a est munie d'une pluralité de logements 21 destinés chacun à recevoir un unique élément médical 50 afin d'éviter tout contact entre deux de ces éléments 50. Les logements 21 sont typiquement disposés en rangées sur la face supérieure du plateau 20. Un logement 21 de plateau possède un fond 21a, formé de la surface supérieure 20a du plateau 20, et une ouverture 21b pour insérer l'élément 50. Chaque logement 21 est délimité latéralement par une cloison 22, perpendiculaire à la face supérieure 20a du plateau 20, qui définit sa forme. Celle-ci peut être quelconque, par exemple circulaire, hexagonale ou rectangulaire, ajustée à la forme et/ou à la dimension des éléments médicaux 50 que le logement 21 est destiné à accueillir. La cloison 22 est pleine, de manière à isoler latéralement chacun des éléments médicaux 50 lorsqu'ils sont disposés dans les logements 21.

La surface interne de la cloison 22, c'est-à-dire la surface orientée vers l'intérieur du logement 21, peut être munie d'au moins un élément de maintien latéral flexible 23, par exemple une patte flexible 23. Cet élément de maintien 23 permet de retenir l'élément médical 50 dans le logement 21 et de limiter ses mouvements latéraux. On réduit ainsi les possibilités de frottement entre l'élément médical 50 et la cloison 22 et le risque de génération de particules. Le caractère flexible des éléments de maintien latéraux 23 permet de pouvoir utiliser ce logement 21 pour différentes dimensions d'éléments 50 et ainsi tenir compte des dispersions de dimensions pouvant exister même lorsque ces éléments sont tous identiques entre eux.

Avantageusement, les logements 21 sont chacun munis d'au moins une butée 24 pour prévenir un contact étendu entre l'élément médical 50 et le fond 21a du logement 21. Cette butée 24 permet de limiter la surface de frottement avec le fond et réduit la génération de particules. La butée peut être constituée par des nervures fines délimitées sur le fond 21a du logement 21, par exemple trois de ces nervures, ou par des appuis ponctuels, par exemple trois de ces appuis.

La face inférieure 20b du plateau 20 présente une pluralité de capuchons 25, le nombre de capuchons dans cette pluralité étant typiquement égale au nombre de logements 21 disposés sur la face supérieure 20a du plateau 20. Les capuchons 25 sont disposés sur la face inférieure 20b de manière à être chacun au droit d'un logement 21. Les capuchons 25 sont ici délimités, similairement aux logements 21, par des parois 26 de manière à présenter des dimensions complémentaires à celles des logements 21 de sorte que, lorsqu'un premier plateau 20 est empilé sur un second plateau, identique au premier plateau 20, les capuchons 25 du premier plateau 20 soient aptes à refermer, par exemple par emboitement, les logements du second plateau qu'ils surplombent (figure 1). On peut également envisager que les parois 26 définissant les capuchons 25 soient parfaitement alignées avec les cloisons 22 définissant les logements 21 de sorte que les logements soient refermés en plaçant, bord contre bord, les parois 26 d'un premier plateau 20 sur les cloisons 22 d'un second 20, directement sous-jacent dans l'empilement : la fermeture d'un logement 21 du second plateau 20 se fait alors par aboutement de la cloison 22 dudit logement 21 et de la paroi 26 du capuchon 25, en vis-à-vis, du premier plateau 20 disposé sur le second plateau 20 (figure 5). La paroi 26 est pleine, de manière à isoler latéralement chacun des éléments médicaux 50 lorsqu'ils sont disposés dans les logements 21 fermés par les capuchons 25 du plateau 20 supérieur.

Chaque capuchon 25 est également défini par un fond 25a, formé par la face inférieure 20b du plateau 20 associé.

Comme on l'a déjà mentionné, les capuchons 25 du plateau inférieur 20' de l'empilement peuvent également permettre d'assembler ce plateau par emmanchement sur les bossages 11 du fond 10a de la cuve 10 (figure 1).

Dans le cas d'une fermeture des logements 21 par emmanchement, les dimensions internes du capuchon 25 sont avantageusement supérieures aux dimensions externes du logement 21 afin de localiser l'éventuelle zone de frottement entre les logements 21 et les capuchons 25 sur la surface externe des cloisons des logements 21, comme cela est bien visible sur la figure 3. Dit autrement, les parois 26 des capuchons 25 encadrent par l'extérieur les cloisons 22 des logements 21 et sont en contact avec la surface externe des cloisons 22. On prévient ainsi que des particules soient générées à l'intérieur des logements 21 lors d'éventuels frottements des parois 26 contre les cloisons 22. Avantageusement, on prévoira un léger jeu latéral, typiquement compris entre 0,1 et 1 mm, entre les parois 26 définissant les capuchons 25 et les cloisons 22 définissant les logements 21 pour empêcher de forcer l'emboitement des plateaux 20 lors de leur empilement, ce qui pourrait rendre difficile l'accès ultérieur aux éléments médicaux retenus dans un plateau. Selon une première variante, illustrée notamment sur les figures 1 et 5, chaque logement 21 présente un fond 21a plein, formé par la face supérieure 20a du plateau 20, et chaque capuchon 25 présente un fond 25a plein, formé par la face inférieure 20b du plateau 20. Lorsque deux plateaux 20 sont empilés, les éléments médicaux 50 (par exemple, des bouchons), disposés dans les logements 21 du plateau 20 sous-jacent, sont totalement isolés les uns des autres et protégés des potentielles contaminations particulaires générées durant le transport du système d'emballage 100 ou lors de son ouverture.

Selon une deuxième variante, illustrée sur les figures 6a et 6b, chaque logement 21 présente un fond 21a ajouré, et chaque capuchon 25 présente également un fond 25a ajouré, du fait de la présence d'un orifice 28 dans le plateau 20, centré sur le fond 21a de chaque logement 21. L'orifice 28 associé à un logement 21 présente alors des dimensions inférieures aux dimensions de l'élément médical 50 destiné à occuper ledit logement 21, de sorte que la partie supérieure extérieure de l'élément médical 50 est apte à obturer l'orifice 28. La partie inférieure de l'élément médical 50 est quant à elle en contact avec le fond 21a du logement 21.

Cette deuxième variante est intéressante en ce qu'elle limite la quantité de matière pour la fabrication d'un plateau 20. Elle est particulièrement adaptée dans le cas d'un élément médical 50 prenant la forme d'un bouchon 51 muni d'une coiffe de verrouillage 52, comme représenté sur les figures 6a et 6b. Le bouchon 51 est habituellement composé d'un matériau élastomère, présentant une tête et un pied destiné (en utilisation) à être inséré dans le col d'un flacon. La coiffe de verrouillage 52 entoure le bouchon 51 et est destinée (en utilisation) à venir s'agripper sous la collerette du flacon, lorsque le pied du bouchon 51 est totalement inséré dans le col : la coiffe de verrouillage 52 comporte des organes de retenue aptes à se bloquer sous la collerette pour sécuriser le bouchon 51 sur le flacon. La coiffe de verrouillage 52 comporte également des moyens assurant le maintien du bouchon 51 dans ladite coiffe 52 lors du stockage et du transport dans le système d'emballage 100. Sans que cela soit limitatif, on pourra trouver des exemples d'éléments médicaux 50 de ce type dans les documents EP2464577 ou EP2464580.

La coiffe de verrouillage 52 de l'élément médical 50 peut comprendre une capsule 52a formant la partie supérieure extérieure dudit élément médical 50. Comme cela est illustré sur les figures 6a et 6b, c'est cette partie supérieure extérieure 52a qui obture l'orifice 28 et permet d'isoler entre eux les éléments médicaux 50 d'une même colonne (c'est-à-dire, à l'aplomb les uns des autres, dans les plateaux 20 empilés).

En particulier, le bouchon 51 de l'élément médical 50, qui constitue la partie la plus sensible en termes de contamination, est totalement isolé dans le logement 21 d'un plateau 20 :
- par l'emboitement ou l'aboutement de la cloison 22 du logement 21 du plateau 20 avec la paroi 26 du capuchon 25 en vis-à-vis d'un plateau supérieur,
- du fait de l'obturation du fond ajouré 25a du capuchon 25 du plateau supérieur par la partie supérieure extérieure 52a de la coiffe de verrouillage 52 de l'élément médical 50 disposé dans le logement 21 du plateau 20, et potentiellement,
- du fait de l'obturation du fond ajouré 21a du logement 21 par la partie supérieure extérieure 52a de la coiffe de verrouillage 52 d'un élément médical 50 disposé dans un plateau sous-jacent au plateau 20.

Dans le cas particulier du plateau inférieur 20', le plus proche du fond 10a de la cuve 10, on pourra obturer l'orifice 28 du fond ajouré 21a des logements 21 dudit plateau 20' en prévoyant des capuchons ou obturateurs complémentaires au niveau du fond 10a de la cuve 10, ou laisser les orifices 28 ouverts.

Selon une troisième variante, qui mixe les première et deuxième variantes précitées, seulement une partie (au moins un) des logements 21 d'un plateau 20 peut présenter un fond 21a ajouré, l'autre partie présentant un fond 21a plein.

Revenant à la description générale, on peut avantageusement prévoir de munir certains au moins des capuchons 25 d'un plateau 20 d'éléments de maintien vertical 27 (visible sur la figure 3 sous la forme d'une languette flexible 27), pour maintenir et limiter le mouvement vertical des éléments médicaux 50 disposés dans les logements du plateau sous-jacents. On réduit ainsi les éventuels frottements liés au mouvement de l'élément médical 50 au sein du logement 21. Tout comme pour les éléments de maintien latéraux 23, le caractère flexible des éléments de maintien verticaux 27 permet de pouvoir utiliser un logement 21 pour différentes dimensions d'éléments 50 et/ou tenir compte des dispersions de dimensions de ces éléments.

De manière avantageuse, la partie supérieure des logements 21, c'est-à-dire l'extrémité supérieure des cloisons 22, est déformable. Dans l'exemple de la figure 3, l'extrémité supérieure des cloisons 22 présente un profil en pointe. Les efforts de compression qui s'exercent sur l'extrémité supérieure des cloisons 22 lors de l'empilement des plateaux 20 conduisent à leur déformation, par écrasement, ce qui permet de compenser les éventuels défauts de planéité des plateaux 20. Cela assure de refermer convenablement tous les logements des plateaux, notamment lors d'une étape ultérieure de mise sous vide de la cuve.

Les plateaux peuvent être composés d'une matière plastique, par exemple à base de polypropylène ou d'élastomère thermoplastique ou encore de polybutylène téréphtalate (PBT).

Chaque plateau 20 comporte avantageusement un rebord périphérique de préhension 20c, qui peut se situer dans le prolongement latéral des faces supérieure 20a et inférieure 20b, comme illustré sur les figures 1, 2 et 3. Alternativement, comme cela est visible sur les figures 5, 6a et 6b, le rebord périphérique de préhension 20c peut se situer dans le prolongement latéral de l'extrémité supérieure des cloisons 22 des logements 21.

### Description du couvercle

On désigne génériquement par « couvercle » tout élément placé dans la cuve 10, sur le plateau supérieur 20'' de l'empilement, pour en refermer les logements. On évite ainsi de laisser vide ce plateau supérieur 20'' de tout élément médical 50, comme cela a été évoqué antérieurement.

Selon un premier mode de réalisation, illustré par la figure 4a, le couvercle 30 peut être une plaque rigide 30 munie sur sa face inférieure d'une pluralité de capuchon 32 aptes à refermer, par exemple par emboitement, les logements 21 du dernier plateau 20". Ces capuchons 32 sont donc identiques, dans leurs formes, dimensions et dispositions sur la face inférieure de la plaque 30, aux capuchons 25 des plateaux 20. La plaque rigide 30 et les capuchons 32 peuvent être formés du même matériau que celui formant les plateaux 20 et la plaque présenter des dimensions similaires.

Dans une variante de ce mode de réalisation, la plaque peut être composée d'une pluralité de bandes 30' individuelles destinées à recouvrir et refermer respectivement des rangées de logements 21 du plateau supérieur 20". Ces bandes 30' peuvent être rendues solidaires de l'opercule poreux 40, par exemple par soudure, de sorte que le retrait de l'opercule 40 (lors de l'ouverture du système d'emballage 100) entraine naturellement l'ouverture des logements 21 du plateau supérieur 20". Dans ce mode de réalisation dans lequel les bandes 30' sont rigides, celles-ci sont préférentiellement disposées perpendiculairement à la direction d'enlèvement de l'opercule poreux 40 afin de faciliter son enlèvement.

Selon un deuxième mode de réalisation, illustré par la figure 4b, le couvercle 30 est composé d'une plaque flexible ou d'une pluralité de bandes flexibles 30' dont la face inférieure est munie de capuchon 32 similaire à ceux des plateaux 20. Tout comme dans le premier mode de mise en œuvre, les bandes 30' sont configurées pour refermer une rangée de logements 21 du plateau supérieur 20" de l'empilement. Pour conférer ou améliorer le caractère flexible d'une bande 30', celle-ci peut être formée de zones de flexion 33, relativement étroites, reliant entre elles des zones de support, relativement larges, portant les capuchons 32.

La bande flexible 30' et les capuchons 32 peuvent être formés d'un matériau souple par exemple à base d'élastomères thermoplastiques.

Ce deuxième mode de réalisation est tout particulièrement avantageux lorsque la plaque flexible 30 ou les bandes 30' sont solidaires de l'opercule 40 qui referme la cuve 10. On ouvre alors les logements 21 du plateau supérieur 20'' de l'empilement disposé dans cette cuve, lors du retrait de cet opercule, et on accède immédiatement aux éléments médicaux 50 stockés dans les logements 21 du plateau supérieur 20''. Si les bandes 30' sont suffisamment flexibles, elles peuvent être disposées sans orientation particulière vis-à-vis du sens d'ouverture de l'opercule 40.

Selon un troisième mode de réalisation, illustré par la figure 4c, on peut également prévoir que le couvercle 30 soit mis en œuvre par une pluralité de capuchons unitaires 30", ces capuchons étant respectivement disposés sur les logements du plateau supérieur 20'' pour les refermer. Il peut s'agir de capuchons souples ou rigides.

### Description de l'opercule

L'opercule poreux 40 est destiné à être scellé, par exemple par soudure plastique, sur les bords supérieurs de la paroi 10b de la cuve 10, une fois que celle-ci a été emplie des plateaux 20 portant les éléments médicaux 50 et éventuellement du couvercle. L'opercule poreux vise à préserver la propreté des éléments médicaux 50 et prévenir l'introduction de particules dans la cuve 10. La porosité à l'air de l'opercule 50 permet d'extraire l'air de la cuve, lors d'une étape suivante de mise sous vide. Il peut par exemple être en Tyvek^{®}, un matériau couramment utilisé dans l'industrie pharmaceutique.

Lorsque l'on prévoit de disposer dans la cuve un couvercle 30 sur le plateau supérieur 20" de l'empilement comme cela a déjà été évoqué en référence aux deux modes de réalisation de ce couvercle 30, l'opercule poreux peux également être scellé, par exemple par soudure, à ce couvercle 30 simultanément à son scellement sur les bords supérieurs de la paroi 10b de la cuve 10.

Pour permettre cela, les éléments du système d'emballage 100 sont dimensionnés de sorte que la surface exposée du couvercle 30 (quel que soit le mode de réalisation retenu) lorsque celui-ci est disposé sur le plateau supérieur 20'' de l'empilement de plateaux, soit affleurant au bord supérieur de la paroi de la cuve 10.

### Description du procédé d'emballage

Afin de réduire au maximum la contamination par des particules et préserver l'éventuel caractère stérile des éléments médicaux 50, les différentes étapes dont la description suit sont préférablement réalisées dans un environnement contrôlé.

Un premier plateau 20', le plateau inférieur 20' de l'empilement, est disposé sur le fond 10a de la cuve 10 de manière à aligner et emmancher les capuchons 25 de la face inférieure 21a du premier plateau 21' avec les bossages 11 du fond 10a de la cuve 10 (s'ils sont présents). Des éléments médicaux 50 sont ensuite ou préalablement disposés dans les logements 21 du premier plateau 20'.

Ensuite, un deuxième plateau 20 est disposé sur le premier plateau 20'. Les capuchons 25 disposés sur la face inférieure du deuxième plateau referment les logements 21 du premier plateau 20'. Des éléments médicaux 50 sont disposés dans chaque logement 21 du deuxième plateau 20, avant ou après cette opération. On répète cela autant de fois qu'il y a de plateaux 20 à déposer dans la cuve 10 et jusqu'à déposer le dernier plateau 20'', formant le plateau supérieur 20'' de l'empilement.

Dans le cas où aucun couvercle 30 n'est prévu, le dernier plateau 20" est maintenu vide, c'est-à-dire sans placer dans ses logements 21 d'éléments médicaux.

Dans le cas contraire, on dépose un couvercle 30 sur la face supérieure 21a du plateau supérieur 20'' de l'empilement afin de refermer ses logements 21 dans lesquels on a préalablement disposé les éléments médicaux. Comme on l'a déjà évoqué, ce couvercle peut être formé d'une plaque munie de capuchons qui est donc disposée d'un seul tenant sur le plateau supérieur 20". Il peut alternativement s'agir d'une pluralité de bandes 30' flexibles ou rigides portant des capuchons, et déposés en rangées sur le plateau supérieur 20" de sorte à refermer les logements 21 ou encore de capuchons unitaires 30'' disposés sur chaque logement 21. Optionnellement, on peut utiliser une combinaison de ces options pour refermer les logements 21 du plateau supérieur 20".

L'opercule poreux 40 est ensuite disposé sur le plateau supérieur 20'' ou sur le couvercle 30, puis solidarisé aux bords supérieurs de la paroi 10b de la cuve 10, ainsi qu'éventuellement à la plaque flexible 30 ou aux bandes 30' et/ou capuchons unitaires 30" lorsque ceux-ci sont présents.

Dans une étape suivante, la cuve 10 dont l'ouverture a été refermée par l'opercule poreux 40 est placée dans au moins une sache étanche à l'air (et préférentiellement deux saches pour des raisons de sécurité), et le vide est réalisé dans cette sache avant de la sceller hermétiquement. La mise sous vide de l'ensemble permet de bloquer verticalement les différents composants de l'assemblage 100 et donc de bien refermer individuellement chaque logement 21. Les éventuels défauts de planéité des plateaux 20 et du couvercle 30 sont compensés par la nature déformable des cloisons 22 des logements 21 et par la souplesse relative des plateaux 20. La mise sous vide permet également de bloquer horizontalement les différents composants de l'assemblage 100 en déformant la paroi périphérique 10b de la cuve 10 pour la plaquer contre la tranche des plateaux 20. La mise sous vide permet ainsi de limiter la friction entre les différents éléments de l'assemblage 100 et donc la génération de particules par friction en bloquant leur mouvement vertical et horizontal.

### Ouverture du système d'emballage

Pour ouvrir le système d'emballage et accéder aux éléments médicaux qu'il conditionne, on extrait tout d'abord de la sache la cuve 10 contenant l'empilement de plateaux 20. L'opercule 40 est ensuite retiré pour exposer le couvercle 30, lorsque celui-ci est présent. Le couvercle 30 est extrait de la cuve, par exemple grâce à une ventouse lorsque celui-ci se présente sous la forme d'une plaque rigide, pour exposer les logements 21 du plateau supérieur 20". Lorsqu'il se présente sous la forme de bandes rigides ou flexibles scellées à l'opercule 30, l'ouverture de cet opercule conduit à naturellement retirer les capuchons des logements 21, sans opération additionnelle. Les éléments médicaux 50 peuvent ensuite être prélevés individuellement ou rangée par rangée, manuellement et/ou automatiquement par une machine. Une fois le plateau supérieur 20" vidé de ses éléments médicaux 50, ce dernier est également retiré pour exposer les logements 21 et les éléments médicaux 50 du plateau 20 sous-jacent. On répète ces opérations jusqu'à prélever tous les éléments médicaux 50 stockés dans la cuve 10.

Bien entendu l'invention n'est pas limitée aux modes de mise en œuvre décrits et on peut y apporter des variantes de réalisation sans sortir du cadre de l'invention tel que défini par les revendications.

Bien que l'on ait ici décrit un seul type de logement délimité par des cloisons, on pourrait envisager d'autres formes ou configurations. Les logements peuvent, par exemple, être disposés en alvéoles pour optimiser la place dans la cuve, la surface interne d'une cloison d'un logement formant la surface externe d'une cloison d'un autre logement. Les logements pourraient également tout aussi bien correspondre à un creux formé dans un plateau ou être une combinaison de creux et de cloisons.

Enfin, bien qu'ici on ait mis en avant un emboitement des capuchons sur les logements s'effectuant par l'extérieur pour localiser la zone de frottement possible sur la surface externe des logements, il est bien sûr envisageable d'avoir l'inverse. Dans ce cas de figure, les dimensions externes du capuchon 25 sont inférieures aux dimensions internes du logement 21 pour que l'emboitement du capuchon 25 s'effectue par l'intérieur.

## Revendications

1. Plateau de livraison (20) d'éléments médicaux (50), le plateau (20) comprenant une face inférieure (20b) et une face supérieure (20a), la face supérieure (20a) présentant une pluralité de logements (21) destinés chacun à recevoir un unique élément médical (50);
le plateau (20) étant **caractérisé en ce que** la face inférieure (20b) du plateau (20) présente une pluralité de capuchons (25), les capuchons (25) étant disposés et dimensionnés de sorte que lorsque le plateau (20) est empilé sur un second plateau identique, chaque capuchon (25) du plateau (20) soit apte à refermer un logement (21) du second plateau.

2. Plateau (20) selon la revendication précédente dans lequel :
- chaque logement (21) est délimité latéralement par une cloison (22) perpendiculaire à la face supérieure (20a) du plateau (20),
- chaque capuchon (25) est délimité latéralement par une paroi (26) perpendiculaire à la face inférieure (20b) du plateau (20), et
- les capuchons (25) du plateau (20) sont respectivement disposés au droit des logements (21) du plateau (20) et sont respectivement dimensionnés pour refermer les logements (21) d'un second plateau identique sous-jacent audit plateau (20), par emboitement ou par aboutement des cloisons (22) des logements (21) du second plateau et des parois (26) des capuchons (25) en vis-à-vis du plateau (20).

3. Plateau (20) selon l'une des revendications précédentes dans lequel les logements (21) présentent chacun une surface interne, la surface interne des logements (21) étant munie d'au moins un élément de maintien latéral (23) pour maintenir et limiter les mouvements latéraux de l'élément médical (50) dans le logement (21).

4. Plateau (20) selon l'une des revendication précédente dans lequel les capuchons (25) sont munis chacun d'un élément de maintien vertical (27) pour maintenir et limiter le mouvement vertical d'un élément médical (50) disposé dans le logement du second plateau sur lequel le plateau (20) a été empilé.

5. Plateau (20) selon l'une des revendications précédentes dans lequel chaque logement (21) présente un fond (21a) plein, formé par la face supérieure (20a) du plateau (20), chaque capuchon (25) présentant également un fond (25a) plein, formé par la face inférieure (20b) du plateau (20).

6. Plateau (20) selon l'une des revendications 1 à 4 dans lequel au moins un logement (21) présente un fond (21a) ajouré, du fait de la présence d'un orifice (28) dans le plateau (20), centré sur le fond (21a) du -au moins un-logement (21), l'orifice (28) présentant des dimensions inférieures aux dimensions de l'élément médical (50) destiné à occuper ledit logement (21).

7. Plateau (20) selon l'une des revendications précédentes dans lequel une partie supérieure des logements (21) est déformable.

8. Système d'emballage (100) pour éléments médicaux (50) comprenant :
- une cuve (10) ayant une ouverture, un fond (10a) et une paroi périphérique (10b) ;
- un empilement formé d'une pluralité de plateaux (20) conformes à l'une des revendications précédentes, l'empilement étant placé dans la cuve (10) ; et
- un opercule poreux (40) scellé sur un bord supérieur de la paroi périphérique (10b) de la cuve (10) pour la refermer.

9. Système d'emballage (100) selon la revendication précédente comprenant en outre un couvercle (30) disposé sur l'empilement de plateaux pour refermer les logements (21) d'un plateau supérieur (20'') de l'empilement.

10. Système d'emballage (100) selon la revendication précédente dans lequel le couvercle (30) est une plaque rigide munie sur une face inférieure d'une pluralité de capuchons (32) aptes à refermer les logements (21) du plateau supérieur (20") de l'empilement.

11. Système d'emballage (100) selon la revendication 9 dans lequel le couvercle (30) est composé d'une pluralité de bandes (30') munies de capuchons (32) aptes à refermer les logements (21) du plateau supérieur (20'') de l'empilement.

12. Système d'emballage (100) selon la revendication précédente dans lequel les bandes (30') et les capuchons (32) sont formées d'un matériau flexible.

13. Système d'emballage (100) selon la revendication précédente dans lequel chaque bande (30') comprend des zones de flexion (33) formées entre chaque capuchon (32).

14. Système d'emballage (100) selon la revendication 8 dans lequel les logements (21) d'un plateau supérieur (20'') de l'empilement sont refermés par des bouchons unitaires (30").

15. Système d'emballage (100) selon l'une des revendications 8 à 14 dans lequel le fond (10a) de la cuve (10) est muni de bossages (11) configurés pour s'emboiter dans des capuchons (25) d'un plateau inférieur (20') de l'empilement.

16. Système d'emballage (100) selon l'une des revendications 8 à 15 dans lequel la cuve (10) est placée, sous vide, dans au moins une sache étanche.

17. Procédé d'emballage d'éléments médicaux (50) **caractérisé en ce qu'**il comprend les étapes suivantes :
- fournir une cuve (10) présentant une ouverture, un fond (10a) et une paroi périphérique (10b) ;
- disposer un plateau inférieur (20') conforme à l'une des revendications 1 à 7 au fond (10a) de la cuve (10);
- disposer au moins un plateau supplémentaire conforme à l'une des revendications 1 à 7 pour former un empilement de plateaux (20) dans la cuve (10), les capuchons (25) du plateau supplémentaire refermant les logements (21) d'un plateau directement sous-jacent ;
- sceller un opercule poreux (40) sur un bord supérieur de la paroi périphérique (10b) de la cuve (10) ;
- placer la cuve (10) dans au moins une sache étanche, et mettre la sache sous vide.

18. Procédé d'emballage selon la revendication précédente dans lequel l'opercule (40) est rendu solidaire de la cuve (10) par soudage de l'opercule (40) sur le bord supérieur de la paroi périphérique de la cuve (10).

19. Procédé d'emballage selon l'une des deux revendications précédentes comprenant une étape visant à disposer un couvercle (30) sur un plateau supérieur (20") de l'empilement.

20. Procédé d'emballage selon la revendication précédente dans lequel l'opercule (40) est également scellé au couvercle (30).

## Patentansprüche

1. Ablagetablett (20) für medizinische Produkte (50), wobei das Tablett (20) eine Unterseite (20b) und eine Oberseite (20a) umfasst, wobei die Oberseite (20a) eine Vielzahl von Aufnahmen (21) aufweist, die jeweils zum Aufnehmen eines einzelnen medizinischen Produkts (50) bestimmt sind;
wobei das Tablett (20) **dadurch gekennzeichnet ist, dass** die Unterseite (20b) des Tabletts (20) eine Vielzahl von Kappen (25) aufweist, wobei die Kappen (25) so angeordnet und dimensioniert sind, dass beim Stapeln des Tabletts (20) auf ein zweites, identisches Tablett jede Kappe (25) des Tabletts (20) dazu geeignet ist, eine Aufnahme (21) des zweiten Tabletts zu verschließen.

2. Tablett (20) nach dem vorhergehenden Anspruch, wobei:
- jede Aufnahme (21) durch eine Trennwand (22) seitlich abgegrenzt ist, die senkrecht zur Oberseite (20a) des Tabletts (20) verläuft,
- jede Kappe (25) durch eine Wand (26) seitlich abgegrenzt ist, die senkrecht zur Unterseite (20b) des Tabletts (20) verläuft, und
- die Kappen (25) des Tabletts (20) jeweils auf Höhe der Aufnahme (21) des Tabletts (20) angeordnet und so dimensioniert sind, dass sie die Aufnahmen (21) eines zweiten, identischen Tabletts, das unter dem Tablett (20) liegt, durch Einrasten oder Aneinanderstoßen der Trennwände (22) der Aufnahmen (21) des zweiten Tabletts und der Wände (26) der Kappen (25) gegenüber dem Tablett (20) verschließen.

3. Tablett (20) nach einem der vorhergehenden Ansprüche, wobei die Aufnahmen (21) jeweils eine Innenfläche aufweisen, wobei die Innenfläche der Aufnahmen (21) mit mindestens einem seitlichen Halteelement (23) versehen ist, um die seitlichen Bewegungen des medizinischen Produkts (50) in der Aufnahme (21) zu halten und zu begrenzen.

4. Tablett (20) nach einem der vorhergehenden Ansprüche, wobei die Kappen (25) jeweils mit einem vertikalen Halteelement (27) versehen sind, um die vertikale Bewegung eines in der Aufnahme des zweiten Tabletts (20) angeordneten medizinisches Produkts (50) zu halten und zu begrenzen.

5. Tablett (20) nach einem der vorhergehenden Ansprüche, wobei jede Aufnahme (21) einen massiven Boden (21a) aufweist, der durch die Oberseite (20a) des Tabletts (20) gebildet wird, wobei jede Kappe (25) ebenfalls einen massiven Boden (25a) aufweist, der durch die Unterseite (20b) des Tabletts (20) gebildet wird.

6. Tablett (20) nach einem der Ansprüche 1 bis 4, bei dem mindestens eine Aufnahme (21) einen perforierten Boden (21a) aufweist, aufgrund des Vorhandenseins einer Öffnung (28) in dem Tablett (20), die auf dem Boden (21a) der -mindestens einen- Aufnahme (21) mittig angeordnet ist, wobei die Öffnung (28) Abmessungen aufweist, die kleiner sind als die Abmessungen des medizinischen Produkts (50), das diese Aufnahme (21) belegen soll.

7. Tablett (20) nach einem der vorhergehenden Ansprüche, wobei ein oberer Teil der Aufnahmen (21) verformbar ist.

8. Verpackungssystem (100) für medizinische Produkte (50), umfassend:
- einen Behälter (10), der eine Öffnung, einen Boden (10a) und eine Umfangswand (10b) aufweist;
- einen Stapel, der aus einer Vielzahl von Tabletts (20) nach einem der vorhergehenden Ansprüche gebildet ist, wobei der Stapel in dem Behälter (10) angeordnet ist; und
- ein poröses Verschlusselement (40), das an einem oberen Rand der Umfangswand (10b) des Behälters (10) versiegelt ist, um ihn zu verschließen.

9. Verpackungssystem (100) nach dem vorhergehenden Anspruch, ferner umfassend einen Deckel (30), der auf dem Stapel von Tabletts angeordnet ist, um die Aufnahmen (21) eines oberen Tabletts (20") des Stapels zu verschließen.

10. Verpackungssystem (100) nach dem vorhergehenden Anspruch, wobei der Deckel (30) eine starre Platte ist, die an ihrer Unterseite mit einer Vielzahl von Kappen (32) versehen ist, die zum Verschließen der Aufnahmen (21) des oberen Tabletts (20") des Stapels geeignet sind.

11. Verpackungssystem (100) nach Anspruch 9, wobei der Deckel (30) aus einer Vielzahl von Streifen (30') besteht, die mit Kappen (32) versehen sind, welche zum Verschließen der Aufnahmen (21) des oberen Tabletts (20") des Stapels geeignet sind.

12. Verpackungssystem (100) nach dem vorhergehenden Anspruch, wobei die Streifen (30') und die Kappen (32) aus einem flexiblen Material gebildet sind.

13. Verpackungssystem (100) nach dem vorhergehenden Anspruch, wobei jeder Streifen (30') zwischen den einzelnen Kappen (32) gebildete Flexzonen (33) aufweist.

14. Verpackungssystem (100) nach Anspruch 8, wobei die Aufnahmen (21) eines oberen Tabletts (20") des Stapels durch einheitliche Stopfen (30") verschlossen sind.

15. Verpackungssystem (100) nach einem der Ansprüche 8 bis 14, wobei der Boden (10a) des Behälters (10) mit Vorsprüngen (11) versehen ist, die so konfiguriert sind, dass sie in Kappen (25) eines unteren Tabletts (20') des Stapels einrasten.

16. Verpackungssystem (100) nach einem der Ansprüche 8 bis 15, wobei der Behälter (10) in mindestens einem undurchlässigen Beutel unter Vakuum platziert ist.

17. Verpackungsverfahren für medizinische Produkte (50),
**dadurch gekennzeichnet, dass** es die folgenden Schritte umfasst:
- Bereitstellen eines Behälters (10), der eine Öffnung, einen Boden (10a) und eine Umfangswand (10b) aufweist;
- Anordnen eines unteren Tabletts (20') nach einem der Ansprüche 1 bis 7 am Boden (10a) des Behälters (10);
- Anordnen mindestens eines zusätzlichen Tabletts nach einem der Ansprüche 1 bis 7, um einen Stapel von Tabletts (20) im Behälter (10) zu bilden, wobei die Kappen (25) des zusätzlichen Tabletts die Aufnahmen (21) eines direkt darunter befindlichen Tabletts verschließen;
- Versiegeln eines porösen Verschlusselements (40) an einem oberen Rand der Umfangswand (10b) des Behälters (10);
- Platzieren des Behälters (10) in mindestens einen undurchlässigen Beutel und Anlegen von Vakuum auf den Beutel.

18. Verpackungsverfahren nach dem vorhergehenden Anspruch, wobei das Verschlusselement (40) mit dem Behälter (10) durch Löten des Verschlusselements (40) an den oberen Rand der Umfangswand des Behälters (10) fest verbunden wird.

19. Verpackungsverfahren nach einem der beiden vorhergehenden Ansprüche, umfassend einen Schritt zum Anordnen eines Deckels (30) auf einem oberen Tablett (20") des Stapels.

20. Verpackungsverfahren nach dem vorhergehenden Anspruch, wobei das Verschlusselement (40) ebenfalls mit dem Deckel (30) versiegelt ist.

## Claims

1. Delivery tray (20) for medical items (50), the tray (20) comprising a lower face (20b) and an upper face (20a), the upper face (20a) having a plurality of recesses (21) each intended to receive a single medical item (50);
the tray (20) being **characterized in that** the lower face (20b) of the tray (20) has a plurality of caps (25), the caps (25) being arranged and dimensioned so that, when the tray (20) is stacked on a second identical tray, each cap (25) of the tray (20) is able to close a recess (21) of the second tray.

2. Tray (20) according to the preceding claim, wherein:
- each recess (21) is delimited laterally by a partition (22) perpendicular to the upper face (20a) of the tray (20),
- each cap (25) is delimited laterally by a wall (26) perpendicular to the lower face (20b) of the tray (20), and
- the caps (25) of the tray (20) are respectively arranged in line with the recesses (21) of the tray (20) and are respectively dimensioned to close the recesses (21) of a second identical tray underlying said tray (20) by nesting or by abutment of the partitions (22) of the recesses (21) of the second tray and of the walls (26) of the caps (25) opposite the tray (20).

3. Tray (20) according to either of the preceding claims, wherein the recesses (21) each have an inner surface, the inner surface of the recesses (21) being provided with at least one lateral support element (23) to support and limit the lateral movements of the medical item (50) in the recess (21).

4. Tray (20) according to any of the preceding claims, wherein the caps (25) are each provided with a vertical support element (27) to support and limit the vertical movement of a medical item (50) arranged in the recess of the second tray on which the tray (20) has been stacked.

5. Tray (20) according to any of the preceding claims, wherein each recess (21) has a solid bottom (21a) formed by the upper face (20a) of the tray (20), each cap (25) also having a solid bottom (25a) formed by the lower face (20b) of the tray (20).

6. Tray (20) according to any of claims 1 to 4, wherein at least one recess (21) has a perforated bottom (21a), due to the presence of an orifice (28) in the tray (20), centered on the bottom (21a) of the - at least one - recess (21), the orifice (28) having dimensions smaller than the dimensions of the medical item (50) that is intended to occupy said recess (21).

7. Tray (20) according to any of the preceding claims, wherein an upper part of the recesses (21) is deformable.

8. Packaging system (100) for medical items (50), comprising:
- a vessel (10) having an opening, a bottom (10a) and a peripheral wall (10b);
- a stack formed by a plurality of trays (20) according to any of the preceding claims, the stack being placed in the vessel (10); and
- a porous lid (40) sealed on an upper edge of the peripheral wall (10b) of the vessel (10) in order to close it.

9. Packaging system (100) according to the preceding claim, further comprising a cover (30), arranged on the stack of trays, to close the recesses (21) of an upper tray (20") of the stack.

10. Packaging system (100) according to the preceding claim, wherein the cover (30) is a rigid plate provided on a lower face with a plurality of caps (32) capable of closing the recesses (21) of the upper tray (20") of the stack.

11. Packaging system (100) according to claim 9, wherein the cover (30) is composed of a plurality of strips (30') provided with caps (32) capable of closing the recesses (21) of the upper tray (20") of the stack.

12. Packaging system (100) according to the preceding claim, wherein the strips (30') and the caps (32) are formed by a flexible material.

13. Packaging system (100) according to the preceding claim, wherein each strip (30') comprises bending regions (33) formed between each cap (32).

14. Packaging system (100) according to claim 8, wherein the recesses (21) of an upper tray (20") of the stack are closed by unitary stoppers (30").

15. Packaging system (100) according to any of claims 8 to 14, wherein the bottom (10a) of the vessel (10) is provided with bosses (11) configured to nest into the caps (25) of a lower tray (20') of the stack.

16. Packaging system (100) according to any of claims 8 to 15, wherein the vessel (10) is placed, under vacuum, in at least one sealed bag.

17. Method for packaging medical items (50), **characterized in that** it comprises the steps of:
- providing a vessel (10) having an opening, a bottom (10a) and a peripheral wall (10b);
- arranging a lower tray (20') according to any of claims 1 to 7 at the bottom (10a) of the vessel (10);
- arranging at least one additional tray according to any of claims 1 to 7 in order to form a stack of trays (20) in the vessel (10), the caps (25) of the additional tray closing the recesses (21) of a directly underlying tray;
- sealing a porous lid (40) on an upper edge of the peripheral wall (10b) of the vessel (10);
- placing the vessel (10) in at least one sealed bag, and vacuumizing the bag.

18. Packaging method according to the preceding claim, wherein the lid (40) is rigidly connected to the vessel (10) by welding the lid (40) onto the upper edge of the peripheral wall of the vessel (10).

19. Packaging method according to either of the two preceding claims, comprising a step aimed at arranging a cover (30) on an upper tray (20") of the stack.

20. Packaging method according to the preceding claim, wherein the lid (40) is also sealed to the cover (30).
